(19)

Europäisches Patentamt
European Patent Office
Office européen des brevets

(11)  **EP 4 102 402 A1**

(12)  **EUROPEAN PATENT APPLICATION**

(43) Date of publication:
**14.12.2022  Bulletin 2022/50**

(21) Application number: **21305776.3**

(22) Date of filing: **08.06.2021**

(51) International Patent Classification (IPC):
**G06K 9/46** *(2006.01)*     **A61B 5/00** *(2006.01)*
**A61N 1/36** *(2006.01)*     **G01R 33/56** *(2006.01)*
**G06K 9/62** *(2022.01)*     **G16H 30/40** *(2018.01)*

(52) Cooperative Patent Classification (CPC):
**G06K 9/6271; A61B 5/0042; A61N 1/37247;**
**G01R 33/5608; G06V 10/454; G16H 30/40;**
**G16H 50/20;** A61N 1/0534; A61N 1/3606;
G01R 33/4822; G01R 33/5602; G06V 2201/031

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Syneika**
**35000 Rennes (FR)**
• **Université de Rennes 1**
**35000 Rennes (FR)**
• **Institut National de la Santé et de la**
**Recherche Médicale (INSERM)**
**75013 Paris (FR)**

(72) Inventors:
• **BAXTER, John**
**35000 RENNES (FR)**
• **BUI, Quoc Anh**
**31000 TOULOUSE (FR)**
• **CROCI, Stéphane**
**35510 CESSON-SEVIGNE (FR)**
• **DELMAS, Antoine**
**35131 CHARTRES DE BRETAGNE (FR)**

(74) Representative: **Plasseraud IP**
**66, rue de la Chaussée d'Antin**
**75440 Paris Cedex 09 (FR)**

(54)  **DEVICE AND METHOD FOR LOCATING TARGET CEREBRAL POINTS IN MAGNETIC RESONANCE IMAGES**

(57)     It is disclosed a device for locating target points on a magnetic resonance image of the brain of a subject, comprising a trained neural network configured to receive as input a 3D MR image of the brain of a subject, and to output the location, on said image, of at least one determined brain target point,

wherein the neural network comprises a plurality of processing stages ($S_i$) arranged such that:

- each processing stage ($S_i$) is configured to process an image ($I_{r_i}$) at a respective resolution ($r_i$),

- the processing stage of lowest resolution is configured for outputting an estimate of the location of each target point, and

- each other processing stage is configured for:

o receiving, from a processing stage of lower resolution, an estimate of the locations of the target points,

o cropping the input image ($I_{r_i}$) to a smaller region surrounding each estimated target point,

o determining an updated estimate of the location of each target point, and providing the updated estimation to the processing stage of the next higher resolution,

wherein the estimate determined by the processing stage of highest resolution forms the output of the neural network.

FIG. 2

**Description**

FIELD OF THE INVENTION

The present disclosure relates to a device and method for locating determined brain target points on a magnetic resonance image of the brain of a subject. This disclosure finds particular application in locating target points for Transcranial Magnetic Stimulation (TMS) or Deep Brain Stimulation.

TECHNICAL BACKGROUND

Transcranial Magnetic Stimulation is a recent and growing therapy for a variety of neurological disorders though to arise from, or be modulated by, cortical regions of the brain represented by singular 3D target points.

These target points are usually determined from pre-operative magnetic resonance images (MR Images) manually. Accordingly, an expert visualizes an anatomical standard MR Image of the brain of the patient and locates the target points on the patient's cortical surface. This manual annotation is however time consuming and requires rare skills.

There is also a growing interest in automatically identifying the target points using a pre-annotated atlas. An atlas is an MRI brain image of a person in which target points have been identified by one or more experts. A brain MRI image of a subject is the deformably registered to the image of the atlas, and the target points identified on the atlas are then projected onto the cortical surface of the subject. Such approach has been discussed for instance in Ahdab R, Ayache SS, Brugières P, Goujon C, Lefaucheur JP. Comparison of "standard" and "navigated" procedures of TMS coil positioning over motor, premotor and prefrontal targets in patients with chronic pain and depression. Neurophysiol Clin. 2010 Mar;40(1):27-36. doi: 10.1016/j.neucli.2010.01.001. Epub 2010 Jan 22. PMID: 20230933.

However, this method is still quite time-consuming, and it is also susceptible to error resulting from topological differences between the atlas image and the particular brain morphology of a patient, due to the high patient variability in the number and position of cortical gyri.

There is therefore a need for an improved solution for locating said brain target points, which is both faster and more reliable than the prior art solutions.

PRESENTATION OF THE INVENTION

In view of the above, one aim of the invention is to alleviate at least part of the inconveniences of the prior art.

In particular, one aim of the invention is to provide a device and a method for locating target points on a magnetic resonance image of the brain of a subject.

Another aim of the invention is to enable performing said localization in less time than the prior art methods, but with acceptable precision.

To this end, it is proposed a device for locating target points on a magnetic resonance image of the brain of a subject, the device comprising a memory storing a trained neural network configured to receive as input a 3D magnetic resonance image of the brain of a subject, and to output the location, on said image, of at least one determined brain target point, and a computer configured for executing said neural network on an input 3D magnetic resonance image of the brain of a subject and, wherein the neural network is a deep convolutional neural network comprising a plurality of processing stages arranged such that:

- each processing stage is configured to process an image at a respective resolution,
- the processing stage of lowest resolution is configured for outputting an estimate of the location of each target point, and
- each other processing stage is configured for:

    ○ receiving, from a processing stage of lower resolution, an estimate of the locations of the target points,
    ○ cropping the input image to a smaller region surrounding each estimated target point,
    ○ determining an updated estimate of the location of each target point, and providing the updated estimation to the processing stage of the next higher resolution,

wherein the estimate determined by the processing stage of highest resolution forms the output of the neural network.

In embodiments, brain target points are cortical target points for Transcranial Magnetic Stimulation, stimulation points for deep brain stimulation, or brain anatomical targets forming reference points for later applications.

In embodiments, the processing stage of highest resolution receives as input the input image, and each other processing stage receives as input an image of lower resolution from the input image obtained by downsampling the input image.

In embodiments, the neural network further comprises, for each processing stage except the processing stage of highest resolution, a pooling layer configured to perform the downsampling of the image that is received as input by the corresponding processing stage.

In embodiments, the neural network is configured for outputting the location of a plurality of target points, and each processing stage comprises a number of convolution layers common to all the target points, and, for each target point, a respective branch configured for computing the location of said target point.

In embodiments, each processing stage further comprises a plurality of residual layers configured to correct the location of each target point computed by each respective branch, wherein the parameters of the residual layers of all processing stages are identical.

In embodiments, the number of processing stages is determined by $1 + \log_2(N/Ns)$ where N is the size of the input image along one dimension and Ns is the size, along one dimension of a cropped region of the image.

According to another aspect, the present disclosure relates to a method implemented by the above device, for locating at least one determined brain target point on a magnetic resonance image of the brain of a subject, the method comprising:

- receiving a 3D magnetic resonance image of the brain of the subject, and
- implementing, on said image, the trained neural network to output the locations on said image of each determined target point.

In embodiments, the 3D magnetic resonance image is a T1-weighted magnetic resonance image.

In embodiments, the method further comprises transmitting the obtained target point locations to a guidance system of a transcranial magnetic simulation device or a deep brain stimulation device.

According to another aspect, the present disclosure relates to a method for training a neural network configured to output, from a 3D resonance magnetic image of a brain, the location of at least one determined brain target point,

wherein the neural network is a deep convolutional neural network comprising a plurality of processing stages arranged such that:

- each processing stage is configured to process an image at a respective resolution,
- the processing stage of lowest resolution is configured for outputting an estimate of the location of each target point, and
- each other processing stage is configured for:

  ○ receiving, from a processing stage of lower resolution, an estimate of the locations of the target points,
  ○ cropping the input image to a smaller region surrounding each estimated target point,
  ○ determining an updated estimate of the location of each target point, and providing the updated estimation to the processing stage of the next higher resolution,

wherein the estimate determined by the processing stage of highest resolution forms the output of the neural network,

and the method comprises providing the neural network with a set of training resonance magnetic images of brains in which the determined target points have been annotated, and, for each training image, computing a loss function quantifying an error between locations computed by neural network and the actual locations of each target points,

wherein the loss function is a weighted sum of the square error of each processing stage of the neural network divided by the resolution of said processing stage.

In embodiments, wherein each weight of the weighted sum is expressed as:

$$ w_l = sigmoid\left(-\alpha\left(\frac{log_2 e_l - log_2 r_l - 1}{log_2 f_l}\right)\right) $$

where $w_l$ is a weight associated to a processing stage l, $f_l$, is the field of view of said processing stage measured in pixels, $e_l$ is the error of said processing stage $r_l$ is the resolution of said processing stage, and $\alpha$ is a fixed parameter.

According to another aspect, the present disclosure relates to a computer-program product comprising code instructions for implementing the methods disclosed above, when they are executed by a computer.

According to another aspect, the present disclosure relates to a non-transitory computer readable storage having stored thereon code instructions for implementing the methods disclosed above, when they are executed by a computer.

According to another aspect, the present disclosure relates to a computer-implemented neural network, characterized in that it is obtained by implementing the training method disclosed above.

According to the proposed device and method, a specific neural network is used to locate determined target points on brain magnetic resonance images. The structure and the training method of the neural network are adapted to the task, providing average errors that are less than the errors of the registration approach and comparable to the errors that exist between individual human experts. However, the time required for locating the positions of the target points is greatly reduced as compared to the prior art solutions.

BRIEF DESCRIPTION OF THE DRAWINGS

Further details, aspects and embodiments of the proposed solution will be described, by way of example, with reference to the drawings.

- Figure 1 schematically shows a device according to an embodiment of the invention,
- Figure 2 schematically represents the structure of a neural network used for locating target points on a brain MR image,
- Figure 3 schematically represents an estimation block of a neural network used for locating target points on a brain MR image.

- Figure 4 schematically represents a residual layer of a neural network used for locating target points on a brain MR image.
- Figure 5 represents comparative errors in the task of locating brain target points on an MR image.
- Figure 6 shows an example of brain MR image annotation of only one target point, as annotated by comparatives methods and experts.
- Figure 7 shows comparative performances of the method according to the disclosure with a prior art registration approach.
- Figure 8 is a chart showing, for different annotation methods or experts, the percentage of points that have been annotated at a correct gyrus.

## DETAILED DESCRIPTION OF AT LEAST ONE EMBODIMENT

### *Description of the device and neural network*

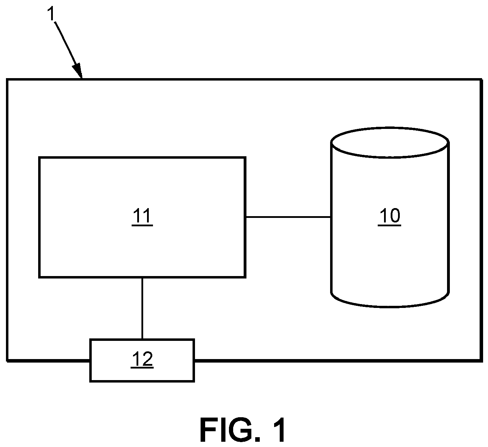

With reference to figure 1, is schematically shown a device 1 for locating target points on a magnetic resonance image of the brain of a subject.

The device 1 comprises a memory 10, storing a trained neural network that is described in greater details below. The memory 10 may store the structure and parameters of the trained neural network as well as code instructions for executing the neural network. The device 1 also comprises a computer 11, configured for executing said neural network on a 3D magnetic resonance image of the brain. The computer may include one or more processor(s), central processing unit(s) and/or graphical processing unit(s).

The computer 11 may receive one or several input image, each input image being a 3D magnetic resonance images of the brain of a subject. To this end, the device 1 may comprise an interface 12 for receiving this image. The interface 12 may include a connection interface with a magnetic resonance imaging device allowing any image acquired by the device to be transmitted to the device 1, either automatically or after performing a selection of one or more images to be transferred. Alternatively the interface 12 may include a connection interface with a memory having stored thereon the image to be processed by the computer 11. According to still other embodiments, the interface 12 may include a connection interface with a communication network, allowing the device to download the input image from the network.

The neural network stored in the memory 10 and executed by the computer 11 is configured for locating, on a 3D magnetic resonance input image of the brain of a subject, a set of determined target points. In embodiments, the input image is a T1-weighted MR image.

The set of determined target points comprises at least one target point, but in embodiments it may comprise a plurality of target points, for instance at least 5 target points, for instance between 5 and 15 target points.

Each target point is defined in three dimensions, the target points and their three dimensions' coordinates forming a cloud of points of interest. In still other embodiments, the target points may be specific brain anatomical targets that can form reference points for later use, for instance, targets allowing the definition of a Talairach space.

Those target points correspond to very specific and punctual locations of the brain, which need to be precisely located in preparation of later neurosurgical intervention. The brain target points can in particular be cortical target points for Transcranial Magnetic Stimulation. They can alternatively be potential stimulation sites for deep brain stimulation.

The neural network is a deep convolutional network configured to process the input image at different resolutions in order to locate the positions of the target points on the input image.

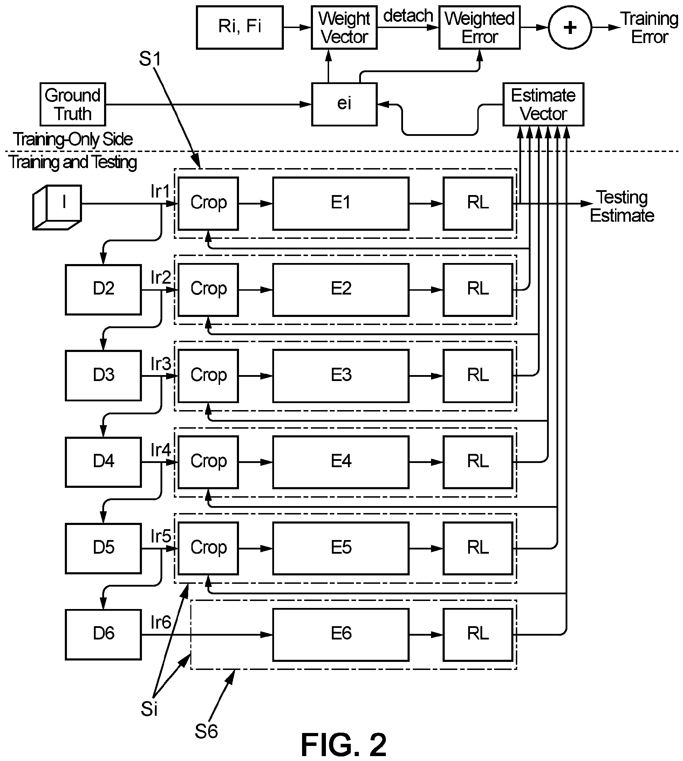

To this end, with reference to figure 2, the neural network comprises a plurality of processing stages $S_i$ (framed by broken lines in figure 2), where each processing stage is configured to process the input image at a respective resolution $r_i$. There is therefore one processing stage $S_1$ of highest resolution, which is configured to process the input image I as received ($I_{r1} = I$), a processing stage of lowest resolution $S_N$ (in figure 2 the image of lower resolution is $I_{r6}$), and there may be one or several additional processing stages processing the input image at respective resolutions which are comprised between the highest ($r_1$ in figure 2) and the lowest resolution ($r_6$ in figure 2).

In the example shown in figure 2, the processing stages are represented with decreasing resolution from the top to the bottom of the figure, the processing stage of highest resolution $S_1$ is thus shown on top and the processing stage of lowest resolution is at the bottom of the figure S6 (the example shown on figure 2 comprises six processing stages).

The processing stage of highest resolution receives as input the input image I in its original resolution ($I_{r1} = I$).

For each other processing stage $S_i$ (i>1), the neural network comprises a downsampling block Di that receives the input image ($I_{ri-1}$) of the processing stage of next higher resolution, and downsamples this image to output an image of lower resolution $I_{ri}$. Said image of lower resolution forms the input image of the considered processing stage $S_i$.

In embodiments, the downsampling block $D_i$ is implemented by an average pooling layer. The pooling size may depend on the size of the original input image. In other embodiments, the downsampling block is implemented by a convolutional layer followed by a ReLU layer (ReLU standing for Rectified Linear Unit).

Therefore, the processing stage of second higher resolution receives as input an image that has been downsampled once, the next stage receives as input an image that has been downsampled twice, etc.

Each processing stage is configured for computing, on its corresponding input image $I_{ri}$, an estimate of the location of each target point of the set of determined target points. Furthermore, each processing stage except the processing stage of lowest resolution is configured to compute said estimate based on an estimate of the location of each target point received from a processing stage of lower resolution, and more specifically from the processing stage of next lower resolution.

Due to the specific function of point localization of the neural network, an important part of the input image does not contribute to solving the problem. Thus, each processing stage Si, except the one of lowest resolution, is further configured to crop its input image, based on the estimate of the location of the target points, to a smaller region of the input image surrounding each estimated target point. This is denoted by a cropping block "crop" at the beginning of each processing stage except the coarsest one, corresponding to the lowest resolution, in figure 2. If the set of determined target points comprises a plurality of target points, the cropping may output an equal number of regions, each surrounding a respective target point.

This cropping operation significantly reduces the requirement for computational memory needed by each processing stage for implementing the subsequent task of locating the target points on its input image. This allows for the processing stages that update the points estimated locations to be relatively large.

In embodiments, each processing stage may thus comprise a plurality of convolutional layers, having a number of kernels that is greater that the number of kernels of the downsampling block Di, if the latter is implemented by a convolutional layer.

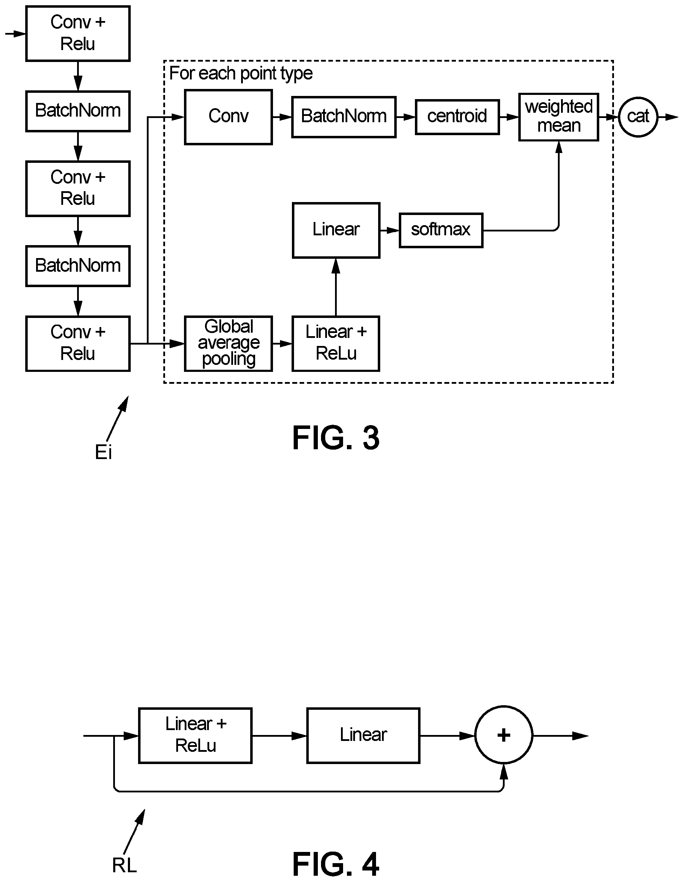

With reference to figure 3 is schematically shown an example of an estimation block Ei of a processing layer, the estimation block being the block receiving the cropped image and outputting an estimate location of each determined target point.

In embodiments, the estimation block of each processing stage comprises one or several layers that are common to all the target points to locate, and then a respective branch configured to compute the location of each target point of the set of predetermined target points.

The layers that are common to all the target points to locate may comprise at least one convolutional layer followed by ReLU layer, followed by a Batch Normalization Layer. In the example shown in figure 3 the layers that are common to all target points comprise two successive blocks composed of convolutional, ReLU and Batch Normalization layer and one additional convolutional and ReLU block.

Furthermore, each branch corresponding to a respective target point (shown in broken line in figure 3) comprises an additional convolutional layer followed by a Batch Normalization layer. The sequence of convolution layers are interpreted as unnormalized probability maps for each point estimate. Thus, to get the updated point estimate, each branch comprises a further block computing a cj defined as:

$$c_{ij} = \frac{\sum_x x e^{M_{ij}(x)}}{\sum_x e^{M_{ij}(x)}}$$

Where j is a channel of the convolutional layer of the respective branch at resolution $r_i$, x a location in the image at resolution $r_i$, Mij(x) is the output of the convolution stack for that channel, and $c_{ij}$ is the centroid of the resulting probability map.

These centroids are treated as potential estimates, and the final updated estimate is taken by a weighted mean of these centroids, where the weight $W_{ij}$ of each channel j is determined by another parallel computation occurring in the same respective branch. This computation comprises an average pooling of the output of the series of layers common to all target points, and a series of two linear layers. An additional softmax layer is applied to normalize these weights.

The final updated estimate is thus given by:

$$s_i = \frac{\sum_j c_{ij} e^{W_{ij}}}{\sum_k e^{W_{ik}}}$$

In embodiments, due to missing or unclear information in the image, it may also be beneficial for these points to be updated, given the knowledge of neighboring similar points. Accordingly, each processing stage of the neural network may further comprise a set of residual layers, comprising a plurality of residual layers RL in series appended to the estimation block $E_i$. With reference to figure 4 is shown one example of residual layer, comprising a summation of the output of the estimation block and of the output of a linear + ReLU layers followed by a linear layer computed on said output.

In order to prevent the residual layers from causing a large increase in the number of parameters, the number of residual layers is the same for all processing stages, and furthermore the parameters of the residual layers are also fixed to be the same for all the processing stages.

Regarding the number of processing stages, this number may vary according to the size of the initial input image and the size of each cropped region. The number NS of processing stages may be computed as:

$$N_S = 1 + \log_2\left(\frac{N}{N_R}\right)$$

Where N is the size of the image along one direction, generally equal to 256, and NR is the size of the cropped image along one direction. This number may be configured. For instance it may be equal to 8. In that case, and as shown in the example of figure 2, the number of layers is 6.

When the trained neural network is applied to an input image, the output of the neural network is formed by the output of the processing stage of highest resolution, which is formed by the input image on which the locations of each determined target point is identified.

*Training*

Back to figure 2, and with reference to the top part of said figure, the training of this neural network will now be disclosed. The training may be implemented by a computer which may be different from the computer of the device executing the trained neural network. The computer used for the training may be a graphical processing unit for instance. This computed also execute code instructions stored on a memory for implementing the training.

The training is performed on a training dataset comprising a plurality of brain MR images which have all been annotated by one or several experts in order to locate, on each image, the position of each point of the set of determined target points. In figure 2, the locations of the target points on the annotated images correspond to the "ground truth" block.

During the training, an error $e_i$ is computed between an estimate vector comprising the estimate output by all the processing stages of the neural network, and the ground truth, corresponding to the positions of the target points determined by the experts. However, given the structure of the network detailed above, it should be underlined that the cropping operations are not differentiable with respect to the location they are cropping around, meaning that the gradients cannot travel from finer resolution layers to coarser ones. Thus conventional gradient-based training function cannot be used. Furthermore, due to the increasing pixel width, changes in the coarser resolution layers are magnified. For instance, an error of 1 pixel at the coarsest layer can be equivalent to an error of 32 pixels at the finest layer.

Last, each layer has a limited space of possible estimates, its so-called "field-of-view", and thus cannot learn from gradients when the ground truth is outside of that space. In order to take into account these considerations, a specific training function is associated to this neural network structure.

Regarding the first problem, an L2 loss can be applied to the estimate from each layer. In other words, the loss function can be expressed as a sum of squared values of the error of each resolution layer.

To address the second problem, the error for each processing stage can be divided by its resolution.

To address the third problem, a weighting scheme is used, configured to take into account, for each processing stage, the error of the processing stage as well as its resolution and field of view, in order to give more importance to processing stages which error has the same order of magnitude as the resolution, and to remove importance from ones in which the error approaches the edges of the field of view or exceeds the field of view.

Thus, the loss function L computed during the training is given by:

$$L = \sum_i w_i \frac{e_i^2}{r_i}$$

Where $r_i$ is the resolution of a processing stage, $e_i$ is the error of this processing stage, and $w_i$ is a weighting coefficient, that can be computed as:

$$w_i = sigmoid\left(-\alpha\left(\frac{\log_2 e_i - \log_2 r_i - 1}{\log_2 f_i}\right)\right)$$

Where $f_i$ is the field of view of a processing stage, and $\alpha$ is a constant that is used to control how close this weighting is to binary, i.e. how much it allows a particular processing stage that is coarser than its optimal resolution but still within its field of view. This value may for instance be set to 0.5.

*Example and results*

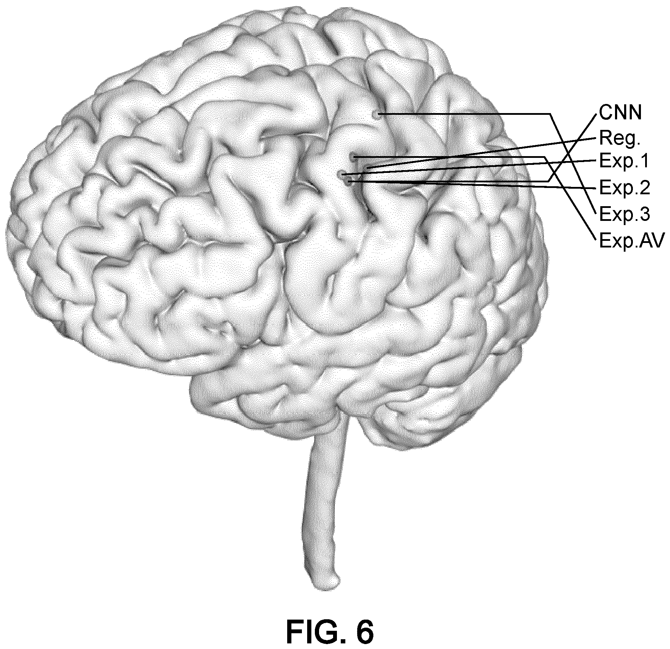

An illustration of the localization of brain points on a MRI images is shown in figure 6. This figure gives one example of a point annotation respectively by :

- the annotation method above, denoted CNN in the figure,
- the registration method approach described in the prior art section, denoted REG in the figure,
- the manual annotation of three experts. The average position of the point (computed by root mean square) is also represented under "Exp. AV" on figure 6.

According to an exemplary embodiment, a neural network having the structure detailed above has been trained and then applied on T1-weighted brain MR images having a size of 256x256x256 pixels.

In this example, the neural network comprises 6 processing stages of respective resolutions. Each downsampling block performs an average pooling of size 2x2x2 pixels. Thus the voxel size processed by the finest resolution layer is 1 mm, and the voxel size processed by the coarsest resolution layer is 32 mm.

Each processing stage except the coarsest one crops its respective input image to a region having a size of 8x8x8 pixels.

The training dataset comprises 16 full volumetric images annotated by experts, on which data augmentation has been implemented in the form of random rotation (std. 10°) and translation (std. 10 pixels), which can be easily applied to the point location as well.

Each image is annotated with 12 determined target points. Preferably, for at least some points, a plurality of experts provides the localization of each point and the location of the point considered as the ground truth is a consensus of the experts on the location. However for some points, a plurality of experts annotations may not be available and the location of a point may be provided by only one expert.

The efficacy of the network is compared to both the locations determined by expert neurologist and to deformable registration performed by deforming an atlas pre-annotated by an expert. The deformable registration procedure takes about 4-5 minutes to compute per patient.

In order to evaluate the method, the mean distance error was calculated in a Leave-One-Out cross validation system, which was repeated 4 times in order to calculate a dispersion metric, that is, an average distance between the point estimate of each network from the mean point across all three other networks.

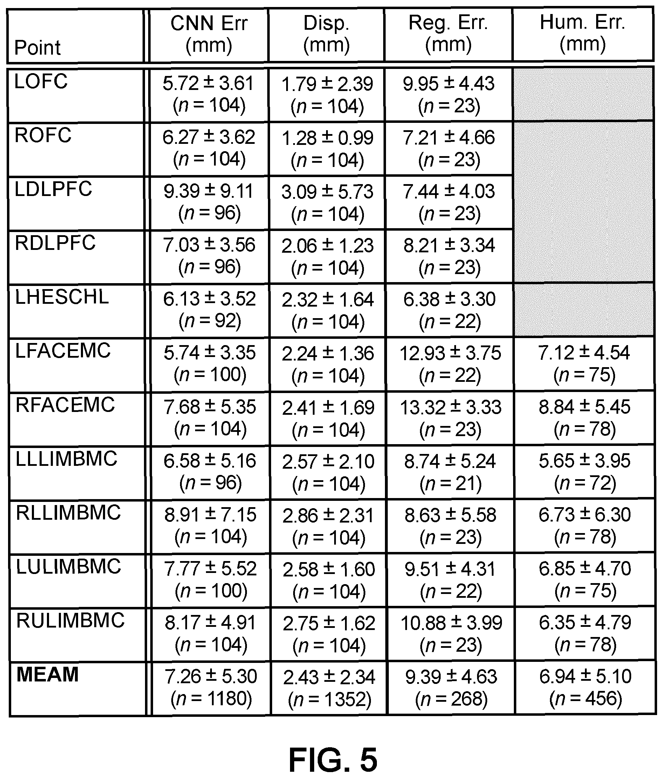

The results are reproduced in figure 5, wherein the "CNN Err" is the error of the disclosed method, "Disp" is its dispersion, "Reg. Err." corresponds to the error of the registration approach and "Hum. Err." is the error between individual human experts and a consensus point. Greyed out cells represent target points for which a consensus point could not be computed due to having only a single expert annotation.

Furthermore, the target points are identified by series of letters, which significations are provided as:

- LOFC: Left Orbitofrontal Frontal Cortex
- ROFC: Right Orbitofrontal Frontal Cortex
- LDLPFC: Left Dorsolateral Prefrontal Cortex
- RDLPFC: Right Dorsolateral Prefontal Cortex
- LHESCHL: Left Heschl's Gyrus
- LFACEMC: Left Primary Motor Cortex (Face)
- RFACEMC: Right Primary Motor Cortex (Face)
- LLLIMBMC : Left Primary Motor Cortex (Lower Limb)
- RLLIMBMC : Right Primary Motor Cortex (Lower Limb)
- LULIMBMC : Left Primary Motor Cortex (Upper Limb)
- RULIMBMC : Right Primary Motor Cortex (Upper Limb)

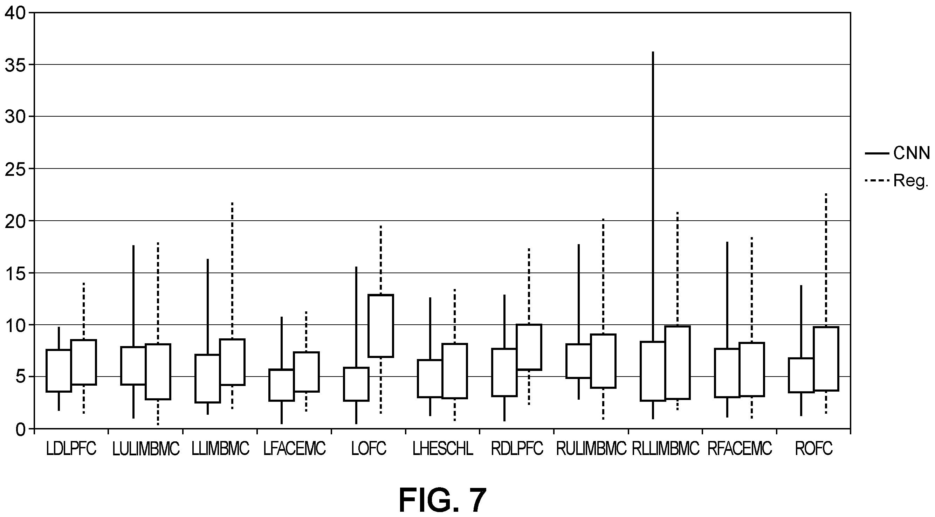

Figure 7 is a chart displaying the average distance in millimeters, for each type of target point, between the point annotated by the disclosed method ("CNN") or the point annotated by the registration approach ("Reg"), and the average location of the point annotated by three experts.

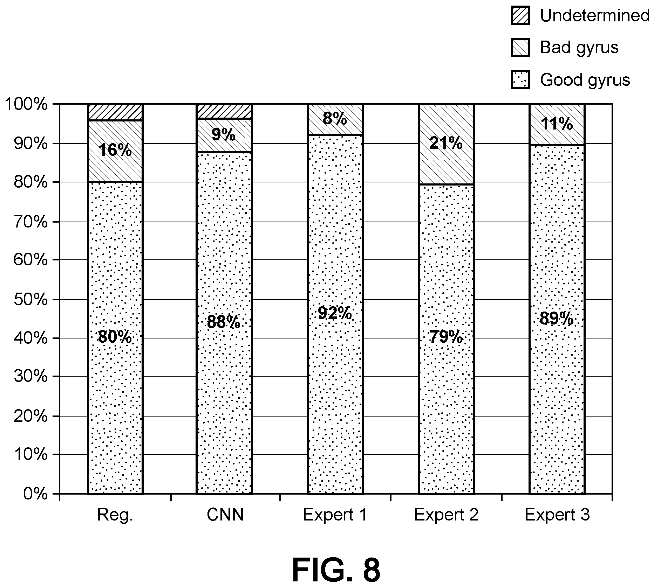

Figure 8 is another chart indicated whether the brain gyrus annotated either by the above method ("CNN"), the registration approach ("Reg") or by three experts is the good gyrus, a erroneous one or whether this aspect is undetermined.

One can thus notice that the proposed method borders on human expert performance and sometimes outperforms human expert error, and sometimes underperforms, but neither to statistical performance. In any case the performance of the method was not achieved by the registration-based approach as the latter consistently underperform the human experts with statistical significance for all but the RLLIMBMC and LULIMBMC target points. Fig. 7 also shows that the above method provides results which are most of the time closer to the average decision provided by human experts than the registration approach, and it is visible from figure 8 that the percentage of good gyrus designated according to the above-disclosed method is higher than the registration approach and even higher than the results achieved by some experts. Furthermore, execution of the neural network on a new input image lasts but a few seconds, whereas the registration approach requires 4 to 5 minutes. The above method thus provides for better performance with drastically reduced computational time.

**Claims**

1. A device (1) for locating target points on a magnetic resonance image of the brain of a subject,
   the device (1) comprising a memory (10) storing a trained neural network configured to receive as input a 3D magnetic resonance image (I) of the brain of a subject, and to output the location, on said image, of at least one determined brain target point, and a computer (11) configured for executing said neural network on an input 3D magnetic resonance image of the brain of a subject and,
   wherein the neural network is a deep convolutional neural network comprising a plurality of processing stages ($S_i$) arranged such that:

   - each processing stage is configured to process an image at a respective resolution ($r_i$),
   - the processing stage of lowest resolution is configured for outputting an estimate of the location of each target point, and
   - each other processing stage is configured for:

     ∘ receiving, from a processing stage of lower resolution, an estimate of the locations of the target points,
     ∘ cropping the input image to a smaller region surrounding each estimated target point,
     ∘ determining an updated estimate of the location of each target point, and providing the updated estimation to the processing stage of the next higher resolution,

wherein the estimate determined by the processing stage of highest resolution ($S^1$) forms the output of the neural network.

2. A device (1) according to claim 1, wherein brain target points are cortical target points for Transcranial Magnetic Stimulation, stimulation points for deep brain stimulation, or brain anatomical targets forming reference points for later applications.

3. A device (1) according to claim 1 or 2, wherein the processing stage of highest resolution ($S_i$) receives as input the input image (I), and each other processing stage receives as input an image ($I_{ri}$) of lower resolution ($r_i$) from the input image (I) obtained by downsampling the input image.

4. A device (1) according to claim 3, wherein the neural network further comprises, for each processing stage except the processing stage of highest resolution, a pooling layer ($D_i$) configured to perform the downsampling of the image that is received as input by the corresponding processing stage.

5. A device (1) according to any of the preceding claims, wherein the neural network is configured for outputting the location of a plurality of target points, and each processing stage ($S_i$) comprises a number of convolution layers common to all the target points, and, for each target point, a respective branch configured for computing the location of said target point.

6. A device (1) according to claim 5, wherein each processing stage further comprises a plurality of residual layers (RL) configured to correct the location of each target point computed by each respective branch, wherein the parameters of the residual layers of all processing stages are identical.

7. A device according to any of the preceding claims, wherein the number of processing stages ($S_i$) is determined by $1 + log_2(N/Ns)$ where N is the size of the input image along one dimension and Ns is the size, along one dimension of a cropped region of the image.

8. A method implemented by the device (1) according to any of the preceding claims for locating at least one determined brain target point on a magnetic resonance image of the brain of a subject, the method comprising:

   - receiving a 3D magnetic resonance image (I) of the brain of the subject, and
   - implementing, on said image, the trained neural network to output the locations on said image of each determined target point.

9. A method according to claim 8, wherein the 3D magnetic resonance image is a T1-weighted magnetic resonance image.

10. A method according to claim 8 or 9, further comprising transmitting the obtained target point locations to a guidance system of a transcranial magnetic simulation device or a deep brain stimulation device.

11. A method for training a neural network configured to output, from a 3D resonance magnetic image of a brain, the location of at least one determined brain target point,
    wherein the neural network is a deep convolutional neural network comprising a plurality of processing stages ($S_i$) arranged such that:

    - each processing stage ($S_i$) is configured to process an image at a respective resolution ($r_i$),
    - the processing stage of lowest resolution is configured for outputting an estimate of the location of each target point, and
    - each other processing stage is configured for:

       ∘ receiving, from a processing stage of lower resolution, an estimate of the locations of the target points,
       ∘ cropping the input image to a smaller region surrounding each estimated target point,
       ∘ determining an updated estimate of the location of each target point, and providing the updated estimation to the processing stage of the next higher resolution,

    wherein the estimate determined by the processing stage of highest resolution ($S_i$) forms the output of the neural network,
    and the method comprises providing the neural network with a set of training resonance magnetic images of brains in which the determined target points have been annotated, and, for each training image, computing a loss function quantifying an error between locations computed by neural network and the actual locations of each target points,
    wherein the loss function is a weighted sum of the square error of each processing stage of the neural network divided by the resolution of said processing stage.

12. A method for training a neural network according to the preceding claim, wherein each weight of the weighted sum is expressed as:

$$w_l = sigmoid\left(-\alpha\left(\frac{log_2 e_l - log_2 r_l - 1}{log_2 f_l}\right)\right)$$

where $w_l$ is a weight associated to a processing stage l, $f_l$, is the field of view of said processing stage measured in pixels, $e_l$ is the error of said processing stage $r_l$ is the resolution of said processing stage, and $\alpha$ is a fixed parameter.

13. A computer-program product comprising code instructions for implementing the method according to any of claims 8-12, when they are executed by a computer (11).

14. A non-transitory computer readable storage having stored thereon code instructions for implementing the method according to any of claims 8-12, when they are executed by a computer (11).

15. A computer-implemented neural network, **characterized in that** it is obtained by implementing the method according to claim 11 or 12.

FIG. 1

FIG. 2

**FIG. 3**

**FIG. 4**

| Point | CNN Err (mm) | Disp. (mm) | Reg. Err. (mm) | Hum. Err. (mm) |
|---|---|---|---|---|
| LOFC | 5.72 ± 3.61 ($n$ = 104) | 1.79 ± 2.39 ($n$ = 104) | 9.95 ± 4.43 ($n$ = 23) | |
| ROFC | 6.27 ± 3.62 ($n$ = 104) | 1.28 ± 0.99 ($n$ = 104) | 7.21 ± 4.66 ($n$ = 23) | |
| LDLPFC | 9.39 ± 9.11 ($n$ = 96) | 3.09 ± 5.73 ($n$ = 104) | 7.44 ± 4.03 ($n$ = 23) | |
| RDLPFC | 7.03 ± 3.56 ($n$ = 96) | 2.06 ± 1.23 ($n$ = 104) | 8.21 ± 3.34 ($n$ = 23) | |
| LHESCHL | 6.13 ± 3.52 ($n$ = 92) | 2.32 ± 1.64 ($n$ = 104) | 6.38 ± 3.30 ($n$ = 22) | |
| LFACEMC | 5.74 ± 3.35 ($n$ = 100) | 2.24 ± 1.36 ($n$ = 104) | 12.93 ± 3.75 ($n$ = 22) | 7.12 ± 4.54 ($n$ = 75) |
| RFACEMC | 7.68 ± 5.35 ($n$ = 104) | 2.41 ± 1.69 ($n$ = 104) | 13.32 ± 3.33 ($n$ = 23) | 8.84 ± 5.45 ($n$ = 78) |
| LLLIMBMC | 6.58 ± 5.16 ($n$ = 96) | 2.57 ± 2.10 ($n$ = 104) | 8.74 ± 5.24 ($n$ = 21) | 5.65 ± 3.95 ($n$ = 72) |
| RLLIMBMC | 8.91 ± 7.15 ($n$ = 104) | 2.86 ± 2.31 ($n$ = 104) | 8.63 ± 5.58 ($n$ = 23) | 6.73 ± 6.30 ($n$ = 78) |
| LULIMBMC | 7.77 ± 5.52 ($n$ = 100) | 2.58 ± 1.60 ($n$ = 104) | 9.51 ± 4.31 ($n$ = 22) | 6.85 ± 4.70 ($n$ = 75) |
| RULIMBMC | 8.17 ± 4.91 ($n$ = 104) | 2.75 ± 1.62 ($n$ = 104) | 10.88 ± 3.99 ($n$ = 23) | 6.35 ± 4.79 ($n$ = 78) |
| **MEAM** | 7.26 ± 5.30 ($n$ = 1180) | 2.43 ± 2.34 ($n$ = 1352) | 9.39 ± 4.63 ($n$ = 268) | 6.94 ± 5.10 ($n$ = 456) |

# FIG. 5

FIG. 6

FIG. 7

FIG. 8

Europäisches
Patentamt

European
Patent Office

Office européen
des brevets

# EUROPEAN SEARCH REPORT

Application Number

EP 21 30 5776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BAXTER JOHN S H ET AL: "Automatic cortical target point localisation in MRI for transcranial magnetic stimulation via a multi-resolution convolutional neural network", INTERNATIONAL JOURNAL OF COMPUTER ASSISTED RADIOLOGY AND SURGERY, SPRINGER, DE, vol. 16, no. 7, 5 June 2021 (2021-06-05), pages 1077-1087, XP037499483, ISSN: 1861-6410, DOI: 10.1007/S11548-021-02386-1 [retrieved on 2021-06-05] * Title Abstract pages 1077-1078, section "Introduction" pages 1080-1081, section "Training page 1078, section "Contributions" pages 1079-1080, sections "Patient images" and "Multi-resolution convolutional neural network architecture"; figure 1 * | 1-15 | INV. G06K9/46 A61B5/00 A61N1/36 G01R33/56 G06K9/62 G16H30/40 |
| A | US 2020/214569 A1 (KIM DONG HYEON [KR]) 9 July 2020 (2020-07-09) * the whole document * | 1,8 | TECHNICAL FIELDS SEARCHED (IPC) G06K G01R G16H A61B A61N G06T |

The present search report has been drawn up for all claims

| Place of search | Date of completion of the search | Examiner |
|---|---|---|
| The Hague | 17 November 2021 | Mukasa, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 21 30 5776

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2021

| Patent document cited in search report | Publication date | Patent family member(s) | Publication date |
|---|---|---|---|
| US 2020214569 A1 | 09-07-2020 | JP 6935630 B2 | 15-09-2021 |
| | | JP 2020533103 A | 19-11-2020 |
| | | KR 20190028900 A | 20-03-2019 |
| | | US 2020214569 A1 | 09-07-2020 |
| | | WO 2019050225 A1 | 14-03-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AHDAB R ; AYACHE SS ; BRUGIÈRES P ; GOUJON C ; LEFAUCHEUR JP.** Comparison of "standard" and "navigated" procedures of TMS coil positioning over motor, premotor and prefrontal targets in patients with chronic pain and depression. *Neurophysiol Clin.,* March 2010, vol. 40 (1), 27-36